# EUROPEAN PATENT SPECIFICATION

(11) **EP 2 918 189 B1**
(45) Date of publication and mention of the grant of the patent: **25.10.2017**
(21) Application number: 15075010.7
(22) Date of filing: 20.02.2015
(51) Int. Cl.: A45C 1/10, A61L 9/04, A61L 9/12, B60R 7/08, B60H 3/00, G07D 9/00

(54) **Scented deodorizing device with pushcart token slot**
PARFÜMIERTE DESODORIERENDE VORRICHTUNG MIT WERTMARKENEINWURF
DISPOSITIF DE DÉSODORISATION PARFUMÉ AVEC FENTE POUR JETONS

(30) Priority: 13.03.2014 IT MI20140095 U
(43) Date of publication of application: 16.09.2015
(73) Proprietor: New Project S.r.l., 20039 Varedo (MB) (IT)
(72) Inventor: Cipolla, Fiorenzo, IT-20815 Cogliate (MB) (IT)
(74) Representative: Riccardi, Sergio

(56) References cited:
- AU-A4- 2011 100 406
- DE-A1-102005 041 560

## Description

The present invention relates to a scented deodorizing device to be used in the interior compartment of a motor vehicle, provided with a token to pick up the pushcart in supermarkets, shopping centers, stores and shops, achieving a considerable usefulness and convenience for users.

It is well known that pushcarts used in the above mentioned businesses, are generally placed in suitable spaces located in the parking areas and/or the business entrance, arranged in aligned rows of carts, each cart being partially inserted into the preceding cart in the row and connected thereto by a chain provided with an end key to be inserted into a corresponding coin box arranged on the handlebar of said preceding cart. In order to release the first cart of the row ready to be picked up, the user should insert a token or coin into the corresponding empty slot of the coin box, thus unlocking the cart chain, and may recover the coin or token only taking the cart back to the parked row and inserting the chain end key into the loaded coin box of the last cart of row, thus locking the returned cart to the parked row and recovering the token or coin at the same time ejected from the coin box.

This action assumes that the user has available a proper token or coin to be inserted into the coin box. Some for-seeing persons always have one such token or coin available inside the vehicle, but there is a high probability that the user lacks such token or coin, thus being compelled either to do without the cart or to look for anybody ready to give him the token or coin. On the other hand many persons are used to keep a deodorizer in the interior compartment of the vehicle, generally placed on the dashboard or air vent or hanging from the rearview mirror.

Document AU 2011 100406 A4 discloses a coin holder comprising a base body with cylindrical cavities, each cavity allowing storage of a plurality of coins, the cavities being provided with a bottom wall to be pressed for taking out the stored coins. These cavities are not provided with a cover to hide the stored coins; moreover to pick up a coin the user must press the bottom wall with one hand and pick up the selected coin with the other hand, and this action is difficult or even impossible for a driver e.g. at the tollgate of a highway or parking lot.

Document DE 10 2005 041560 A1 discloses a coin holder for motor vehicles comprising a plurality of slots provided with opposite internal holding means that make difficult the extraction of the coins blocked between said means.

The present invention brilliantly solves the above mentioned problem, providing for a deodorizer which is also used as a case for said token or coin allowing to pick up the pushcart in supermarkets and like commercial businesses.

For this purpose, the scented deodorizing device of the invention is provided with a slot for the token, which slightly protrudes from the device in such a way that allows to be taken out and used to pick up the cart, and to be inserted again into the slot when the user comes back to the vehicle with the token recovered from the cart.

In this way the user has no more to be careful about having a token or coin available, since the token is always ready in its slot in the vehicle.

The device according to the present invention may be produced in any suitable material, and more particularly impregnated with a deodorizing and/or scenting substance. Materials particularly suitable for said device are EVA (ethylenevinyl acetate) and PP (polypropylene) polymers. As hereinbefore mentioned, the device may be applied on the dashboard or air vent or hanging from the rearview mirror.

The device according to the present invention may have any shape adapted for the intended use, provided that it has an opening allowing to insert into and take out from the slot the token easily, but avoiding that the token comes out from the opening unintentionally, and for this reason the opening should generally be turned upwards. If the user lost the token, a coin of the same size may be inserted in the slot, thus allowing nevertheless to pick up the pushcart.

As a non limiting illustrative example of the invention, an embodiment of the device in the shape of a snail is shown in the accompanying drawings, in which:

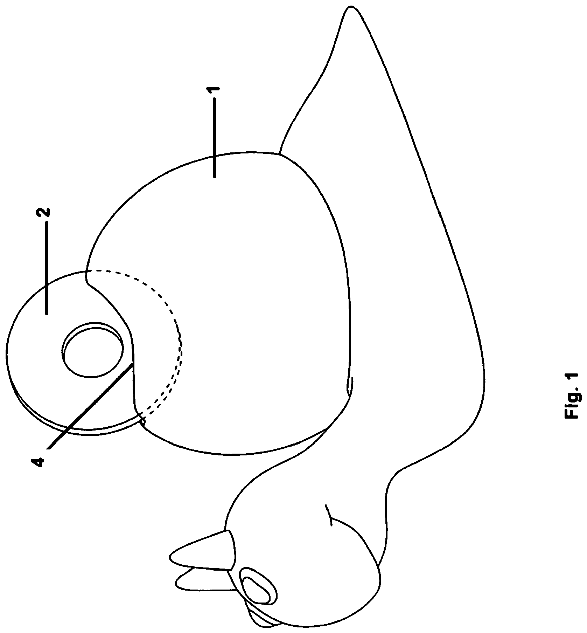

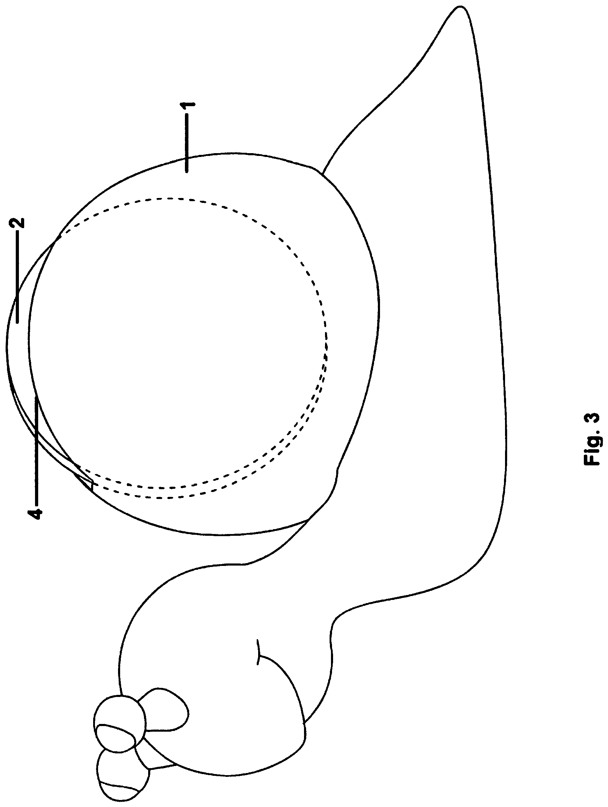

Figure 1 is a side view of the article 1, with the token 2 almost fully taken out from its slot 3 so as to be clearly visible, however keeping in mind that the token 2 normally is received inside the slot 3 and protrudes from the opening 4 of slot 3 only with an upper curved portion of its edge, allowing to take it out, so as to seem like an extension of the upper curve of the snail's shell, thus not allowing an external observer to realize that it is a token or coin contained in the slot 3, as additionally shown in Figure 3.

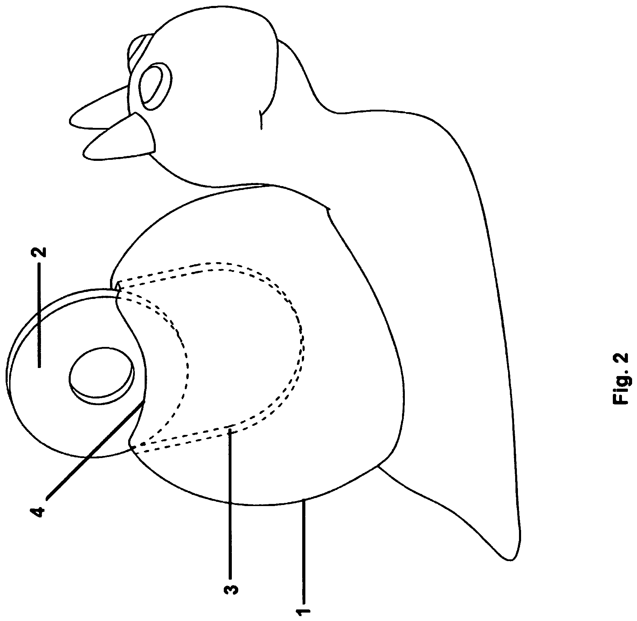

Figure 2 is another side view of the article 1, taken from the opposite view relative to Figure 1, where the approximate depth of the slot 3 is shown in dotted lines.
Figure 3 is a side view similar to Figure 1 of another article 1 of slightly modified design, showing the token 2 inserted into its slot 3, protruding out from the opening 4 just with an upper curved portion of its edge for allowing it to be taken out from said slot 3.

Is it to be pointed out that the device may have any desired shape, the embodiment in the form of snail being only a non limiting example, and the device may have the shape of any other animal, for instance a crocodile, a domestic, farmyard or work animal, or any other article having a shape adapted to be provided in its upper part with an opening of a slot having a depth allowing to receive almost entirely the token or coin to be used for picking up a push cart.

The device is provided with means (not shown) suitable for its arrangement in the interior compartment of a motor vehicle, such as suction cups, magnets, clips or hooks for application e.g. on the dashboard, air vent or sun visors, or a wire or cord for hanging the device on the rearview mirror.

Is it to be understood that materials, shapes and colors of the device do not limit its various embodiments, whose inventive characteristic consists of a scented deodorizing device for motor vehicles, provided with a slot for a token or coin to be used for picking up the pushcart used in supermarkets and like businesses, without departing from the scope of the invention, as defined in the appended claims.

## Claims

1. A scented deodorizing device for the interior compartment of a motor vehicle comprising an article (1) made of a material impregnated with a deodorant substance, and **characterized by** having a single slot (3) for a token (2) or a coin for a supermarket pushcart, whereby said single slot (3) allows for an insertion of said token (2) or coin, leaving just an upper curved portion of its edge protruding out of the slot (3), for allowing it to be taken out from said slot (3).

2. The device of claim 1, **characterized by** being provided with means for its application on the dashboard, the air vents or sun visors of the motor vehicle, such as suction cups, magnets, clips, or hooks.

3. The device according to claim 1, **characterized by** being provided with a wire or cord for hanging to the rearview mirror of the motor vehicle.

4. The device according to claim 1, wherein the material impregnated with the deodorizing or scenting substance is ethylenevinyl acetate (EVA) or polypropylene (PP).

5. The device according to claim 1, **characterized in that** the device has the shape of an animal or any other article (1) suitable to be provided with an opening (4) of the slot (3), said slot (3) having such a depth that the token (2) or coin inserted in the opening (4), protrudes only with an upper curved portion of its edge.

6. The device according to claim 5, wherein the device (1) has the shape of a snail, with the opening (4) of the slot (3) for the token (2) or coin, arranged on the upper curve of the snail's edge.

7. The device according to claim 5, wherein the device has the shape of a crocodile, a domestic, farmyard or work animal having a shape provided with an opening in its upper part, of a slot having a depth allowing to receive almost entirely the token or coin.

## Patentansprüche

1. Parfümierte desodorierende Vorrichtung zur Verwendung im Fahrzeuginnenraum, enthaltend einen aus einem mit einem Desodoriermittel imprägnierbaren Werkstoff bestehenden Gegenstand (1), **gekennzeichnet durch** einen einzigen Schlitz (3) für eine Wertmarke (2) bzw. Münze, um einen bei Geschäften und Supermärkten verfügbaren Einkaufwagen zu entnehmen, worin dieser einzige Schlitz (3) den Einwurf der Wertmarke (2) bzw. Münze erlaubt, wobei nur ein gekrümmtes Oberteil ihres Randes hervorsteht, was genügt, um die Wertmarke bzw. Münze aus dem Schlitz (3) zurückzunehmen.

2. Vorrichtung nach Anspruch 1, **gekennzeichnet durch** Mittel, die für die Anbringung an das Armaturenbrett, die Düsen bzw. die Sonnenblenden des Fahrzeuges, wie Saugnäpfe, Magnete, Klips bzw. Häkchen, geeignet sind.

3. Vorrichtung nach Anspruch 1, **gekennzeichnet durch** einen Faden bzw. eine Schnur für die Aufhängung an den Rückspiegel des Fahrzeuges.

4. Vorrichtung nach Anspruch 1, **dadurch gekennzeichnet, dass** der mit dem Desodoriermittel bzw. parfümierten Mittel imprägnierte Werkstoff Äthylenvinylacetat (EVA) oder Polypropylen (PP) ist.

5. Vorrichtung nach Anspruch 1, **dadurch gekennzeichnet, dass** die Vorrichtung die Form eines Tieres bzw. beliebigen Gegenstands (1) hat und geeignet ist, um einen Eingang (4) des Schlitzes (3) zu schaffen, wobei der Schlitz (3) eine solche Tiefe aufweist, dass die durch den Eingang (4) eingeworfene Wertmarke (2) bzw. Münze nur mit einem gekrümmten Oberteil ihres Randes hervorsteht.

6. Vorrichtung nach Anspruch 5, worin die Vorrichtung (1) die Form einer Schnecke hat, worin der Eingang (4) des Schlitzes (3) für die Wertmarke (2) bzw. Münze an der oberen Krümmung des Schneckenrückens angeordnet ist.

7. Vorrichtung nach Anspruch 5, worin die Vorrichtung die Form eines Krokodils, eines Haustiers, eines Aufzuchttiers, eines Arbeitstiers hat, mit einer Gestalt seines Oberteils, die einen Eingang eines Schlitzes aufweist, wobei der Schlitz eine Tiefe hat, welche die Wertmarke bzw. Münze fast völlig aufnimmt.

## Revendications

1. Dispositif de déodorisation parfumé pour l'habitacle d'un véhicule automobile comprenant un objet (1) fait d'une matière imprégnable avec un agent déodorisant, **caractérisé par** une fente unique (3) pour un jeton (2) ou une pièce pour prendre un chariot utilisé dans les magasins ou supermarchés, dont la dite fente unique (3) permet l'introduction du jeton (2) ou de la pièce que fait saillie de la fente (3) seulement avec une partie supérieure courbe du bord pour prendre le jeton ou la pièce par la fente (3).

2. Dispositif de déodorisation selon la revendication 1, **caractérisé en ce qu'**il comprend des moyens aptes pour l'application à la planche de bord, aux bouches d'air ou aux pare-soleil du véhicule, tels que ventouses, aimants, clips ou crochets.

3. Dispositif selon la revendication 1, **caractérisé en ce qu'**il comprend un fil ou cordonnet pour la suspension au rétroviseur intérieur du véhicule.

4. Dispositif selon la revendication 1, **caractérisé en ce que** la matière imprégnée par l'agent déodorisant ou parfumé est EVA (éthylène-acétate de vinyle) ou polypropylène (PP).

5. Dispositif selon la revendication 1, **caractérisé en ce que** le dispositif a la forme d'un animal ou autre objet (1) quelconque, apte pour être muni par une ouverture (4) de la fente (3), dont la fente (3) a une telle profondeur que le jeton (2) ou la pièce introduit(e) dans l'ouverture (4) fait saillie seulement avec une partie supérieure courbe du bord.

6. Dispositif selon la revendication 5, dont le dispositif (1) a la forme d'un escargot, où l'ouverture (4) de la fente (3) pour le jeton (2) ou la pièce est disposée sur la courbe supérieure du dos de l'escargot.

7. Dispositif selon la revendication 5, dont le dispositif a la forme d'un crocodile, d'un animal domestique, d'élevage ou de travail, ayant une conformation de la partie supérieure, laquelle est munie d'une fente avec une profondeur permettant d'accueillir presque entièrement le jeton ou la pièce.
